# EUROPÄISCHE PATENTSCHRIFT

(11) **EP 2 898 913 B1**
(45) Veröffentlichungstag und Bekanntmachung des Hinweises auf die Patenterteilung: **03.08.2022**
(21) Anmeldenummer: 14198837.8
(22) Anmeldetag: 18.12.2014
(51) Int. Cl.: A61M 5/20, A61M 5/34, A61M 5/315

(54) **Spritze**
Nozzle
Seringue

(30) Priorität: 27.01.2014 DE 102014201393
(43) Veröffentlichungstag der Anmeldung: 29.07.2015
(73) Patentinhaber: Henke-Sass, Wolf GmbH, 78532 Tuttlingen (DE)
(72) Erfinder: Altermann, Frank, 78532 Tuttlingen (DE); Raidt, Simon, 78573 Wurmlingen (DE)
(74) Vertreter: Patentanwälte Geyer, Fehners & Partner mbB

(56) Entgegenhaltungen:
- WO-A1-01/55681
- WO-A1-95/04563
- WO-A1-2010/115670
- JP-A- 2009 011 481
- US-A- 2 129 675
- US-A- 2 375 711
- US-A- 2 551 902
- US-A- 3 248 950
- US-A- 4 946 441
- US-A1- 2004 162 528
- US-A1- 2007 225 656

## Beschreibung

Die vorliegende Erfindung betrifft eine Spritze mit den Merkmalen des Oberbegriffs des Anspruches 1.

Solche Spritzen sind z. B. aus der WO 01/55681 A1 bekannt und können beispielsweise im veterinärmedizinischen Bereich eingesetzt werden. Hier besteht häufig die Anforderung, ein Medikament bei einer Vielzahl von Tieren nacheinander zu applizieren, wobei unterschiedliche Ausspritzmengen benötigt werden.

Die WO 2010/115670 A1 zeigt eine Spritze mit einem Grundkörper, der einen Spritzenzylinder mit einem ausspritzseitigen Ende aufweist, und mit einer Kolbenstange, deren vorderer Endabschnitt in dem Spritzenzylinder entlang einer Verschieberichtung verschiebbar angeordnet ist, wobei der Grundkörper einen vorderen Anschlag für die Kolbenstange, bei der der Abstand zwischen dem vorderen Endabschnitt und dem ausspritzseitigen Ende des Spritzenzylinders minimal ist, aufweist.

Es ist daher Aufgabe der Erfindung, eine Spritze der eingangs genannten Art so weiterzubilden, dass unterschiedliche Ausspritzmengen leicht einstellbar sind.

Erfindungsgemäß wird die Aufgabe bei einer Spritze der eingangs genannten Art dadurch gelöst, dass der Grundkörper eine Führungsrille (die auch als Führungsnut bezeichnet werden kann) für das federnde Druckstück aufweist, die sich entlang der Verschieberichtung erstreckt und in der das federnde Druckstück beim Verschieben des Einstellelementes relativ zum Grundkörper entlang der Verschieberichtung geführt ist.

Mit der erfindungsgemäßen Spritze kann somit in einfacher Art und Weise der maximale Kolbenstangenhub und somit die Ausspritzmenge eingestellt werden. Durch die Führung des federnden Druckstücks in der Führungsrille, wenn die Ausspritzmenge eingestellt bzw. verstellt wird, kann sicher verhindert werden, dass fehlerhafte Einstellungen durchgeführt werden, selbst wenn erschwerte Bedingungen bei der Bedienung vorliegen, weil z. B. gerade die Ausspritzmenge für die Impfung von Tieren in einem Stall zu ändern ist.

Damit kann wiederholt in sicherer Art und Weise die eingestellte Ausspritzmenge appliziert werden.

Weiterbildungen der erfindungsgemäßen Spritze sind in den abhängigen Ansprüchen angegeben.

Insbesondere kann die Spritze als Selbstfüllerspritze ausgebildet sein. Unter einer Selbstfüllerspritze wird insbesondere eine Spritze verstanden, bei der das zu applizierende Fluid (z.B. Medikament) bei einer Bewegung der Kolbenstange vom vorderen Anschlag zum hinteren Anschlag in den Spritzenzylinder gefüllt wird und bei der entgegengesetzten Bewegung vom hinteren Anschlag zum vorderen Anschlag über das ausspritzseitige Ende aus dem Spritzenzylinder ausgespritzt wird.

Bei der erfindungsgemäßen Spritze kann das Einstellelement entlang der Verschieberichtung relativ zum Grundkörper bewegbar sein.

Insbesondere kann das Einstellelement hohlzylinderförmig mit einem Boden ausgebildet sein, wobei der Boden den hinteren Anschlag bildet.

Der Boden kann eine Durchgangsbohrung für die Kolbenstange aufweisen. Die Kolbenstange kann einen seitlichen Vorsprung (z.B. ringförmig) aufweisen, der an einer Innenseite des Bodens anliegt, wenn die Kolbenstange am hinteren Anschlag ist. Der Kontakt zwischen dem Vorsprung und der Innenseite des Bodens bildet somit den hinteren Anschlag.

Die Kolbenstange kann einstückig oder auch mehrstückig sein. Im Bereich der Kolbenstange, der im Spritzenzylinder hin und her bewegt wird, kann auch als Kolben oder Kolbenabschnitt bezeichnet werden.

Bei der erfindungsgemäßen Spritze kann für jede vorgegebene Einstellposition eine Justierrille (bzw. Justiernut) von der Führungsrille quer zur Führungsrille abzweigen, in die das federnde Druckstück durch ein relatives Verdrehen von Einstellelement und Grundkörper bringbar ist. Insbesondere weist jede Justierrille eine Rastvertiefung auf, so dass in Zusammenwirkung mit dem federnden Druckstück die gewünschte Arretierung erzeugt wird.

Diese Arretierung kann durch ein entgegengesetztes Verdrehen gelöst werden, wenn die aufgewandte Kraft so groß ist, dass das federnde Druckstück aus der Rastvertiefung gelöst wird.

Anstatt eines federnden Druckstücks sind auch andere Fixierelemente möglich. Beispielsweise kann ein Verklemmen durch eine Schraube durchgeführt werden, die im Einstellelement geführt ist und gegen den Grundkörper drückt.

Das Einstellelement kann zwei Fixierelemente aufweisen, die bevorzugt einander gegenüberliegend angeordnet sind. Dabei kann es sich beispielsweise um zwei federnde Druckstücke handeln. Für jedes Fixierelement kann die beschriebene Führungsrille sowie die beschriebenen Justierrillen vorgesehen sein. Ferner ist es möglich, dass das Einstellelement ein Fixierelement (wie z.B. in ein federndes Druckstück oder eine Schraube) und ein Führungselement, wie z.B. einen Stift aufweist, die bevorzugt einander gegenüberliegend angeordnet sind. Für das Führungselement kann in gleicher Weise wie für das Fixierelement eine Führungsrille vorgesehen sein. Ferner können am Grundkörper abzweigende Teilführungsrillen vorgesehen sein, die im wesentlichen den Justierrillen entsprechen, aber keine Rastvertiefung aufweisen müssen.

Bei der erfindungsgemäßen Spritze kann die Position des vorderen Anschlags nicht änderbar sein. Dies ist insbesondere bei der Ausbildung als Selbstfüllerspritze sehr vorteilhaft, da das Befüllen des Spritzenzylinders über das ausspritzseitige Ende durchführbar ist und stets das gesamte Fluid aus dem Spritzenzylinder ausgespritzt werden kann.

Die erfindungsgemäße Spritze kann auch als Injektionsvorrichtung und/oder Injektionssystem bezeichnet werden. Weitere Elemente, die für den Betrieb eines solchen Injektionssystems notwendig sind, sind dem Fachmann bekannt und können vorgesehen sein. Insbesondere kann die Kolbenstange z.B. pneumatisch von einem Druckluftzylinder angetrieben werden. Dazu kann das vom Spritzenzylinder wegweisende Ende der Kolbenstange mit dem Druckluftzylinder mechanisch verbunden sein.

Die erfindungsgemäße Spritze kann eine Kanüle aufweisen, die mit dem ausspritzseitigen Ende des Spritzenzylinders in Fluidverbindung steht, so dass das Fluid aus dem Spritzenzylinder über die Kanüle applizierbar ist.

Die Kanüle kann lösbar am Grundkörper befestigt sein. Insbesondere kann die lösbare Verbindung so ausgebildet sein, dass zum Lösen der Kanüle diese nicht in die Hand genommen werden muß.

Dazu kann die Kanüle einen ersten Fluidkanal und eine erste Kontaktfläche aufweisen. Des weiteren kann der Grundkörper der Spritze ein Verbindungsteil mit einem zweiten Fluidkanal und einer zweiten Kontaktfläche aufweisen. Das Verbindungsteil kann durch einen Abschnitt des Grundkörpers selbst oder als separates, mit dem Grundkörper verbundenes Teil ausgebildet sein.

Eine der beiden Kontaktflächen kann als Innenfläche und die andere der beiden Kontaktflächen kann als Außenfläche, die komplementär zur Innenfläche ist, ausgebildet sein. Des weiteren kann eine Aufnahmehülse (beispielsweise Aufnahmemutter) mit einem ersten Gewindeabschnitt und einem ersten Verbindungsmittel für die Kanüle vorgesehen sein. Die Kanüle kann ein zweites Verbindungsmittel aufweisen, das mit dem ersten Verbindungsmittel so zusammenwirkt, dass die Kanüle lösbar mit der Aufnahmehülse verbunden ist. Das Verbindungsteil kann ein zum ersten Gewindeabschnitt komplementären zweiten Gewindeabschnitt aufweisen und durch Verschrauben der beiden Gewindeabschnitte können die beiden Fluidkanäle miteinander verbunden werden und können die erste Kontaktfläche der Kanüle dichtend in Kontakt mit der zweiten Kontaktfläche des Verbindungsteils gebracht werden. Die Aufnahmehülse ist so ausgebildet, dass bei Lösen der Verschraubung der beiden Gewindeabschnitte eine Kraft auf die Kanüle ausgeübt wird, die die erste Kontaktfläche von der zweiten Kontaktfläche wegbewegt. Somit kann das gewünschte Lösen der Kanüle durchgeführt werden.

Insbesondere kann eines der beiden Verbindungsmittel eine Haltenut und das andere der beiden Verbindungsmittel einen Vorsprung aufweisen, der im verschraubten Zustand in die Haltenut einsteht.

Insbesondere kann die Aufnahmehülse eine Durchgangsbohrung mit einer Innenwandung aufweisen, wobei das erste Verbindungsmittel die Haltenut aufweist, die in der Innenwandung ausgebildet ist.

Das zweite Verbindungsmittel kann zwei, drei, vier oder mehr voneinander beabstandete Vorsprünge aufweisen, die jeweils in einer Richtung radial zum ersten Füllkanal vorstehen.

Ferner kann die Aufnahmehülse an ihrem dem Verbindungsteil abgewandten ersten Ende für jeden Vorsprung eine Ausnehmung aufweisen, die sich vom ersten Ende bis zur Haltenut erstreckt, so dass die Kanüle über die Ausnehmung in die Haltenut einsetzbar ist.

Eine der beiden Kontaktflächen kann als Innenkegelfläche und die andere der beiden Kontaktflächen kann als Außenkegelfläche ausgebildet sein.

Die Kanüle kann einen Sockelabschnitt aufweisen, an dem das erste Verbindungsmittel ausgebildet ist.

Insbesondere kann die Verbindung als Luer-Lock-Verbindung ausgebildet sein.

Die Aufnahmehülse kann als Aufnahmemutter ausgebildet sein, bei der der erste Gewindeabschnitt ein Innengewindeabschnitt ist.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine perspektivische Ansicht einer ersten Ausführungsform der erfindungsgemäßen Spritze;
- Fig. 2: eine perspektivische Explosionsdarstellung der Spritze 1 von Fig. 1;
- Fig. 3: eine Schnittdarstellung gemäß der Schnittlinie A-A in Fig. 2;
- Fig. 4: eine Längsschnittdarstellung der erfindungsgemäßen Spritze 1, bei der der hintere Anschlag 21 in einer ersten vorgegebenen Einstellposition ist;
- Fig. 5: eine Schnittdarstellung gemäß Fig. 4, bei der der hintere Anschlag 21 in einer zweiten vorgegebenen Einstellposition ist;
- Fig. 6: eine Schnittdarstellung gemäß Fig. 5 einer Abwandlung der erfindungsgemäßen Spritze 1;
- Fig. 7: eine Draufsicht der Mutter 54 der erfindungsgemäßen Spritze 1;
- Fig. 8: eine Schnittansicht der Aufnahmemutter 54 entlang der Schnittlinie B-B in Fig. 7;
- Fig. 9: eine Schnittansicht der Aufnahmemutter 54 entlang der Schnittlinie C-C in Fig. 7;
- Fig. 10: eine Draufsicht der Kanüle 13;
- Fig. 11: eine vergrößerte Schnittdarstellung der Kanüle 13, der Aufnahmemutter 54 und des Adapters 12 der erfindungsgemäßen Spritze 1, und
- Fig. 12: eine Schnittansicht gemäß Fig. 11, wobei die Kanüle 13 um ihre Längsachse um 90° verdreht ist gegenüber der Position gemäß Fig. 11.

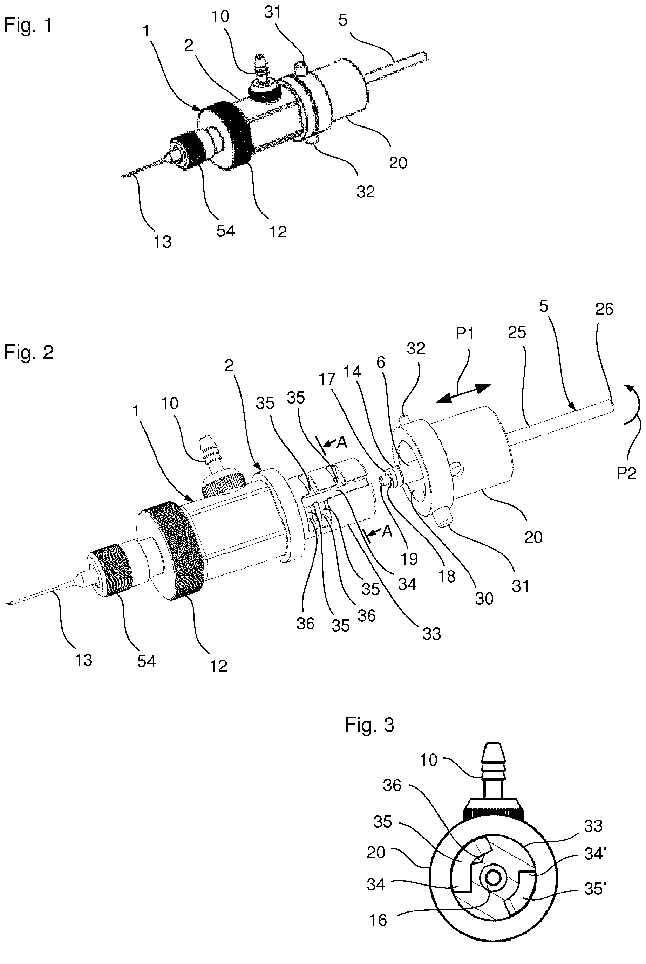

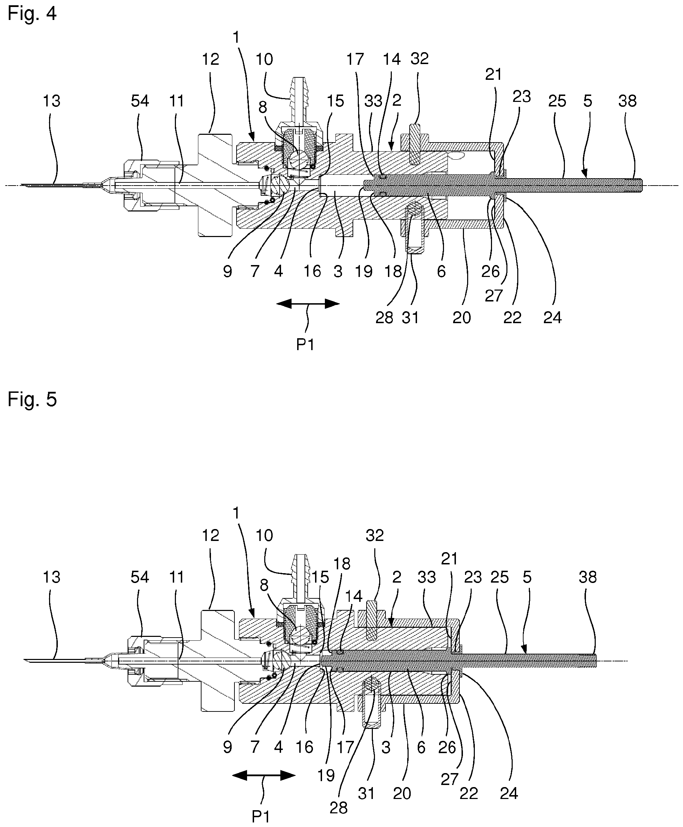

Bei der in Fig. 1-5 gezeigten Ausführungsform umfaßt die erfindungsgemäße Spritze 1 einen Grundkörper 2, in dem ein Spritzenzylinder 3 mit einem ausspritzseitigen Ende 4 (Fig. 4 und 5) ausgebildet ist.

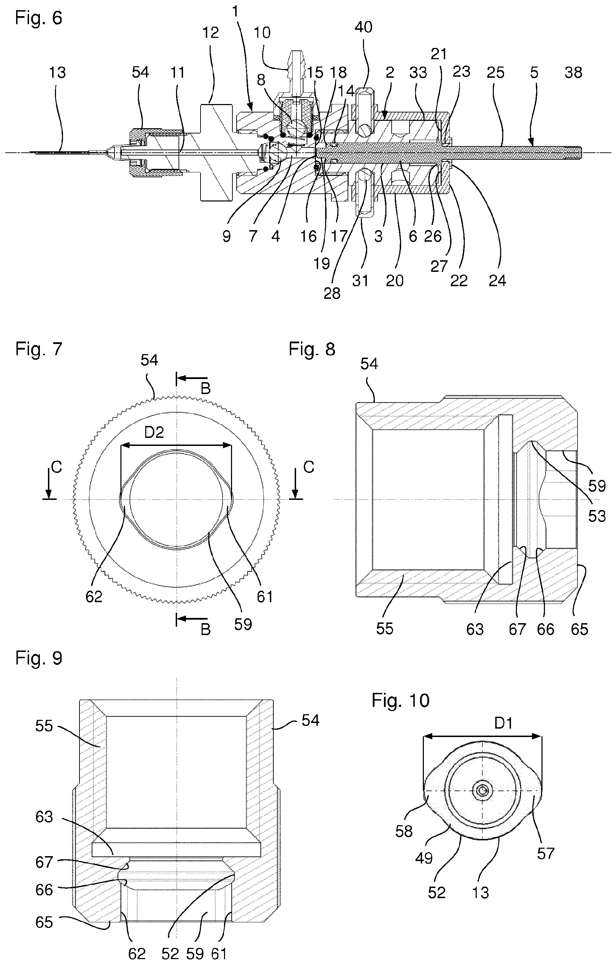

Ferner umfaßt die Spritze 1 eine Kolbenstange 5, deren vorderer Endabschnitt 6 im Spritzenzylinder 3 entlang einer Verschieberichtung P1 (in Fig. 5 und 6 von links nach rechts) verschiebbar angeordnet ist.

Das ausspritzseitige Ende 4 des Spritzenzylinders 3 mündet im Grundkörper 2 in einem T-förmigen Kanalabschnitt 7, in dessen Enden, die nicht mit dem ausspritzseitigen Ende 4 verbunden sind, ein erstes und zweites Rückschlagventil 8, 9 angeordnet sind. Das erste Rückschlagventil 8 ist so ausgebildet, dass bei einem Zurückziehen der Kolbenstange 5 (also einer Bewegung von links nach rechts in Fig. 4 und 5) ein Fluid, wie z.B. ein zu applizierendes Medikament, über einen Fluidanschluß 10 und das erste Rückschlagventil 8 in den Spritzenzylinder 3 fließt bzw. gesaugt wird. Das Fluid kann insbesondere eine Flüssigkeit sein. Das zweite Rückschlagventil 9 ist so ausgebildet, dass bei einer entgegengesetzten Bewegung der Kolbenstange 5 (also von rechts nach links in Fig. 4 und 5) das im Spritzzylinder 3 befindliche Fluid über das zweite Rückschlagventil 9 in einen Kanal 11 eines Adapters 12 und von diesem durch eine mit dem Adapter 12 verbundene Kanüle 13 ausgespritzt wird.

Die erfindungsgemäße Spritze 1 ist so ausgebildet, dass der maximale Hub der Kolbenstange 5 und somit die Menge des abzugebenden Fluids einstellbar ist. Dazu weist der Grundkörper 2 einen vorderen Anschlag 15 für die Kolbenstange 5 auf, der durch Schultern 16 am ausspritzseitigen Ende 4 des Spritzenzylinders 3 gebildet ist. Die Schultern 16 sind dadurch gebildet, dass der Durchmesser des Spritzenzylinders 3 größer ist als der Durchmesser des anschließenden Kanalteils des T-förmigen Kanalabschnitts 7.

Der vordere Endabschnitt 6 der Kolbenstange 5 weist an seinem vorderen Ende 17 eine ringförmige Anschlagfläche 18 auf, die an den Schultern 16 anliegt, wenn der vordere Endabschnitt 6 vollständig in den Spritzenzylinder 3 eingeschoben ist. Bei der hier beschriebenen Ausführungsform weist das vordere Ende 17 noch einen zylinderförmigen Vorsprung 19 auf, der dann in den entsprechenden Teil des T-förmigen Kanalabschnittes 7 einsteht. In einer alternativen Ausführungsform (nicht gezeigt) ist der Vorsprung 19 nicht vorgesehen, so dass das vordere Ende 17 z.B. plan ausgebildet sein kann und in seinem äußeren Bereich die ringförmige Anschlagfläche 18 aufweist. Ferner weist die Kolbenstange 5 im Bereich des vorderen Endabschnitts 6 einen Dichtring 14 auf, der in einer entsprechenden Ringnut sitzt. Wenn die ringförmige Anschlagfläche 18 an den Schultern 16 anliegt, ist der Abstand zwischen dem vorderen Ende 17 und dem ausspritzseitigen Ende 4 minimal.

Die Spritze 1 weist ferner ein hülsenförmiges Einstellelement 20 auf, das einen hinteren Anschlag 21 für die Kolbenstange 5 festlegt, bei dem der Abstand zwischen dem vorderen Ende 17 und dem ausspritzseitigen Ende 4 des Spritzenzylinders 3 maximal ist. Dazu ist im Boden 22 des einen U-förmigen Querschnitt aufweisenden Einstellelementes 20 eine Durchgangsbohrung 23 ausgebildet, durch die die Kolbenstange 5 hindurchläuft. Bei der hier beschriebenen Ausführungsform ist in der Durchgangsbohrung 23 eine Führungshülse 24 eingesetzt, die eine Öffnung im Boden 22 definiert, die koaxial zum Spritzenzylinder 3 ist und dabei einen geringeren Durchmesser als der Spritzenzylinder 3 aufweist.

Die Kolbenstange 5 ist so ausgebildet, dass der vordere Endabschnitt 6 einen an den Spritzenzylinder 3 angepaßten Außendurchmesser aufweist und somit als Kolben dient. An den vorderen Endabschnitt 6 schließt sich ein hinterer Abschnitt 25 der Kolbenstange 5 an, der einen geringeren Durchmesser als der vordere Endabschnitt 6 aufweist. Der Durchmesser des hinteren Abschnitts 25 ist an die durch die Führungshülse 24 definierte Öffnung angepaßt. Ferner weist der vordere Endabschnitt 6 an seinem dem vorderen Ende 17 abgewandten Ende, an das der hintere Abschnitt 25 der Kolbenstange 3 anschließt, einen im wesentlichen ringförmigen Vorsprung 26 auf, der in den Querschnittsdarstellungen gemäß Fig. 4 und 5 an einer Innenseite 27 des Bodens 22 anliegt. Die Innenseite 27 bildet somit den hinteren Anschlag 21 für die Kolbenstange 5, da ein Herausziehen der Kolbenstange 5 aus dem Spritzenzylinder 3 über diese Position nicht möglich ist.

Das Einstellelement 20 ist, wie nachfolgend im Detail beschrieben wird, in Verschieberichtung P1 der Kolbenstange 5 relativ zum Grundkörper 2 verschiebbar und an vorgegebenen Einstellpositionen mit dem Grundkörper 2 fixier- bzw. arretierbar, so dass dadurch die Position des hinteren Anschlags 21 in Verschiebrichtung P1 einstellbar und veränderbar ist. Bei der erfindungsgemäßen Spritze 1 ist der vordere Anschlag 15 fixiert bzw. ist seine Position nicht veränderbar, während der hinter Anschlag 21 verstellbar ist. Mit dieser Art der Einstellung der Position des hinteren Anschlages 21 wird der maximale Hub der Kolbenstange 5 und somit die maximale Fluidmenge festgelegt, die mit einer einzelnen Kolbenstangenbewegung (Verschieben der Kolbenstange 5 von ihrer hinteren Anschlagposition zu ihrer vorderen Anschlagposition) ausspritzbar ist.

Wie in Fig. 4 und 5 ersichtlich ist, weist das Einstellelement 20 im Bereich seines vorderen offenen Endes 30 ein federndes Druckstück 31 und einen Stift 32 auf, die sich jeweils in radialer Richtung erstrecken und einander gegenüberliegend angeordnet sind. Das federnde Druckstück 31 weist an seinem nach innen vorstehenden Ende eine Kugel 28 auf, die durch eine nicht gezeigte Feder mit einer Kraft in Richtung zum Spritzenzylinder 3 hin beaufschlagt ist und zur Arretierung des Einstellelementes 20 dient. Der Stift 32 und das federnde Druckstück 31 sind so im vorderen offenen Ende 30 angeordnet, dass sie über eine Innenwandung des Einstellelementes 20 nach innen vorstehen. Der Durchmesser der Innenwandung des Einstellelementes 20 entspricht dem Außendurchmesser des Grundkörpers 2 in seinem hinteren Endabschnitt 33, in dem der Spritzenzylinder 3 ausgebildet ist. Um eine Verschiebbarkeit des Einstellelementes 20 relativ zum Grundkörper 2 bzw. auf dem hinteren Endabschnitt 33 des Grundkörpers 2 zu ermöglichen, ist sowohl für das federnde Druckstück 31 als auch für den Stift 32 eine sich in Verschieberichtung erstreckende Führungsrille 34, 34' auf der Außenseite des hinteren Endabschnitts 33 ausgebildet. In der perspektivischen Explosionsdarstellung gemäß Fig. 2 ist die Führungsrille 34 für das federnde Druckstück 31 ersichtlich. In der Schnittdarstellung in Fig. 3 (Schnitt entlang A-A in Fig. 2) sind beide Führungsrillen 34, 34' erkennbar. Von der Führungsrille 34 sowie von der Führungsrille 34' gehen jeweils vier Justierrillen 35, 35' ab, die sich jeweils in Umfangsrichtung des hinteren Endabschnittes 33 entlang eines vorbestimmten Winkelbereiches erstrecken. Die Justierrillen 35 für das federnde Druckstück 31 enden jeweils in einer Rastvertiefung 36. Bei dem hier beschriebenen Ausführungsbeispiel erstrecken sich zwei der vier Justierrillen 35 im Uhrzeigersinn und die anderen zwei Justierrillen 35 entgegengesetzt zum Uhrzeigersinn. Gleiches trifft auf die Justierrillen 35' zu. In Verschieberichtung (Doppelpfeil P1 in Fig. 2, 4 und 5) sind die vier Justierrillen 35 und die vier Justierrillen 35' jeweils voneinander beabstandet, so dass vier vorgegebene Einstellpositionen vorhanden sind. Die in Fig. 2 nicht sichtbare Führungsrille 34' für den Stift 32 sowie die entsprechenden Justierrillen 35' weisen jedoch aufgrund des geringeren Durchmessers des Stifts 32 im Vergleich zum federnden Druckstück 31 eine geringere Breite aufweisen. Ferner enden die Justierrillen 35' für den Stift 32 nicht in einer Rastvertiefung.

In der Schnittansicht gemäß Fig. 3 ist eine Justierrille 35 gezeigt, die mit einer Rastvertiefung 36 endet. Die entsprechende Justierrille 35' für den Stift 32 endet ohne Rastvertiefung.

Zur Einstellung eines gewünschten maximalen Kolbenstangenhubs ist ausgehend von der Explosionsdarstellung in Fig. 2 das Einstellelement 20 um 90° gegen den Uhrzeigersinn (Drehrichtung gemäß Pfeil P2 in Fig. 2) zu drehen, so dass das nach innen vorstehende Ende (mit der Kugel 28) des federnden Druckstücks 31 entlang der Führungsrille 34 bewegt werden kann. Das Einstellelement 20 ist dann entlang der Verschieberichtung P1 so zu bewegen, dass das federnde Druckstück 31 auf der Höhe der gewünschten Justierrille 35 liegt und dann wird das Einstellelement 20 durch Drehung in Richtung zu der Rastvertiefung 36 der gewählten Justierrille 35 (also entweder im Uhrzeigersinn oder entgegen des Uhrzeigersinns) arretiert, da bei Erreichen der Rastvertiefung 36 die Kugel 28 des federnden Druckstücks 31 in die Rastvertiefung 36 gedrückt wird.

Somit kann erfindungsgemäß der maximale Kolbenstangenhub eingestellt werden. Die Spritze 1 weist somit verschiedene Rastpositionen für den hinteren Anschlag 22 auf.

Bei der hier beschriebenen Ausführungsform wird die Kolbenstange 5 pneumatisch von einem nicht gezeigten Druckluftzylinder angetrieben. Die entsprechende Verbindung kann mit dem von dem Grundkörper 2 weg weisenden Ende 38 des hinteren Abschnitts 25 der Kolbenstange 5 erfolgen. Bei der beschriebenen Ausführungsform ist am Ende 38 der Kolbenstange 5 ein Außengewinde ausgebildet.

In Fig. 6 ist eine Schnittansicht gemäß Fig. 4 und 5 in einer Abwandlung der erfindungsgemäßen Spritze 1 gezeigt. Die Spritze 1 gemäß Fig. 6 unterscheidet sich von der bisher beschriebenen Spritze 1 darin, dass anstatt des Stiftes 32 ein zweites federndes Druckstück 40 vorgesehen ist. Die entsprechende Führungsrille 34' sowie die entsprechenden Justierrillen 35' sind daher in gleicher Weise wie für das federnde Druckstück 31 der bisher beschriebenen Ausführungsform ausgebildet. Insbesondere weisen die Justierrillen 35' für das zweite federnde Druckstück 40 jeweils eine Rastvertiefung auf. Die restliche Ausbildung der Spritze 1 gemäß Fig. 6 entspricht der Ausbildung der Spritze der Fig. 1 bis 5, so dass gleiche Elemente mit gleichen Bezugszeichen bezeichnet sind und zu deren Beschreibung auf die obigen Ausführungen verwiesen wird.

Bei den Ausführungsformen gemäß Fig. 1 bis 6 ist die Kanüle 13 lösbar am Adapter 12 befestigt, wobei der Adapter 12 lösbar (über eine Schraubverbindung) mit dem Grundkörper 2 verbunden ist. Die Verbindung zwischen Kanüle 13 und Adapter 12 wird nachfolgend in Verbindung mit Fig. 7-12 beschrieben und ist als sogenannte Luer-Lock-Verbindung ausgebildet, wobei der Adapter 12 an seinem dem Spritzenzylinder 3 abgewandten Ende einen Außenkegelabschnitt 50 aufweist, auf dem ein entsprechender Innenkegelabschnitt 51 eines Sockels 52 der Kanüle 13 sitzt. Ein ringförmiger Vorsprung 49 des Sockels 52 der Kanüle 13 sitzt in einer ringförmigen Haltenut 53 einer Aufnahmemutter 54, die auf den Adapter 12 aufgeschraubt ist. Dazu weist die Aufnahmemutter 54 einen Innengewindeabschnitt 55 auf und ist am Adapter 12 ein entsprechender Außengewindeabschnitt 56 ausgebildet.

Wie insbesondere der Draufsicht der Kanüle 13 in Fig. 10 zu entnehmen ist, ist der Umriß des ringförmigen Vorsprungs 49 des Fußes 52 im wesentlichen kreisförmig und weist zwei gegenüberliegende seitliche Vorsprünge 57, 58 auf, so dass dadurch eine Abweichung von der Kreisform vorliegt.

Die Haltenut 53 ist an einer Innenseite einer Durchgangsbohrung 59 im Boden 60 der im Querschnitt im wesentlichen U-förmigen Aufnahmemutter 54 ausgebildet (Fig. 8, 9, 11, 12). Der Bereich der Durchgangsbohrung 59, der von dem dem Adapter 12 abgewandten vorderen Ende 65 der Aufnahmemutter 54 bis zur Haltenut 53 verläuft, weist im Querschnitt einen Umriß auf, der dem Umriß des ringförmigen Vorsprungs 49 der Kanüle 13 entspricht. Wie insbesondere in der Draufsicht von Fig. 7 ersichtlich ist, ist der Umriß der Durchgangsbohrung 59 im wesentlichen kreisförmig und weist zwei radial nach außen vorstehende Ausnehmungen 61, 62 auf. Diese Ausnehmungen 61 und 62 der Durchgangsbohrung 59 erstrecken sich jedoch nur vom vorderen Ende 65 bis zur Haltenut 53. Im Bereich von der Haltenut 53 bis zur dem Adapter 12 zugewandten Innenseite 63 des Bodens 60 sind die Ausnehmungen 61 und 62 nicht ausgebildet, so dass hier die Durchgangsbohrung 59 lediglich eine kreisförmige Querschnittsform aufweist.

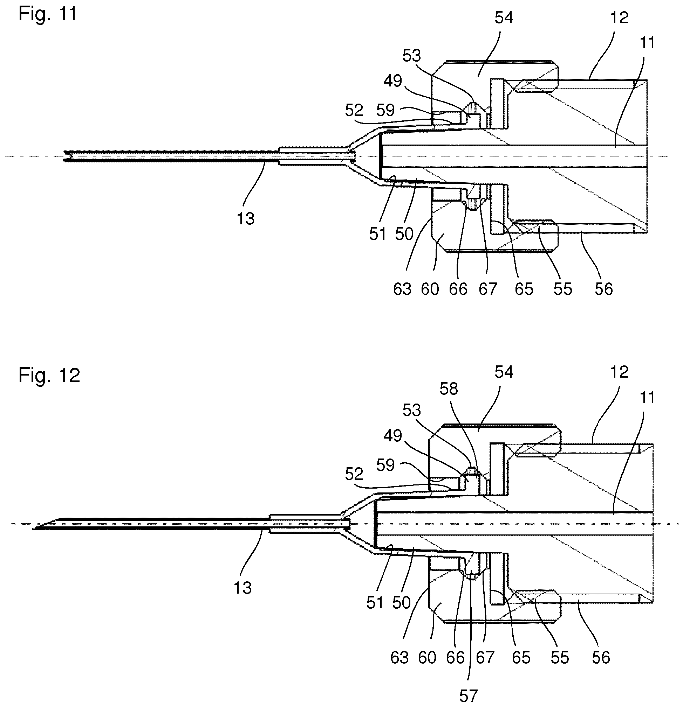

Die Durchgangsbohrung 59 mit den Ausnehmungen 61 und 62 ist so ausgebildet, dass der ringförmige Vorsprung 49 der Kanüle 13 vom vorderen Ende 65 der Aufnahmemutter 54 bis zur Haltenut 53 eingeführt werden kann. Sobald der ringförmige Vorsprung 49 so in die Haltenut 53 eingeführt ist (Fig. 11), kann die Kanüle 13 relativ zur Aufnahmemutter 54 um die Längsachse der Kanüle 13 gedreht werden, wodurch die Vorsprünge 57 und 58 in einem Bereich in der Haltenut 53 positioniert werden, in dem keine Ausnehmungen 61 und 62 ausgebildet sind, so dass die Kanüle 13 in der Haltenut 53 sitzt (Fig. 12).

Bevorzugt ist die maximale Ausdehnung D1 des ringförmigen Vorsprungs, die durch die beiden Vorsprünge 57 und 58 festgelegt ist, so gewählt, dass sie etwas größer ist als die maximale, durch die Ausnehmungen 61, 62 vorhandene lichte Weite D2 der Durchgangsbohrung 59. In diesem Fall muß die Kanüle 13 zum Einsetzen in die Aufnahmemutter 54 leicht gekippt werden (gegenüber der Zeichenebene von Fig. 7). Damit ist es möglich, trotz der größeren Ausdehnung D1 der Vorsprünge 57 und 58 den ringförmigen Vorsprung 49 der Kanüle 13 durch die Ausnehmungen 61, 62 bis zur Haltenut 53 in die Aufnahmemutter 54 einzuführen. Die Dicke der Haltenut 53 (Ausdehnung von links nach rechts in Fig. 8) ist dabei so gewählt, dass der Sockel 52 und somit auch der ringförmige Vorsprung 49 der Kanüle 13 in der Haltenut 53 wieder zurück gekippt werden kann (es liegt somit ein ausreichendes Spiel zwischen dem ringförmigen Vorsprung 49 und der Haltenut 53 vor), so dass selbst ohne Verdrehen der Kanüle 13 relativ zur Aufnahmemutter 54 schon eine gewisse Haltefunktion durch die Haltenut 53 bereitgestellt wird.

Beim Aufschrauben der Aufnahmemutter 54 mit eingesetzter Kanüle 13 liegen die Vorsprünge 57 und 58 an der vorderen Seite 66 der Haltenut 53 an, so dass dadurch der Innenkegelabschnitt 51 der Kanüle 13 auf den Außenkegelabschnitt 50 des Adapters 12 gedrückt wird. Durch den flächigen Kontakt der beiden Kegelabschnitte 50, 51 wird eine Dichtwirkung erreicht. Somit wird die gewünschte Fluidabdichtung zwischen dem Kanal 11 und der Kanüle 13 realisiert, und ein bestimmungsgemäßer Gebrauch der Spritze 1 ist möglich.

Wenn die Kanüle 13 ausgetauscht werden soll, muß lediglich die Aufnahmemutter 54 abgeschraubt werden. Bei diesem Abschrauben drückt dann die hintere Seite 67 der Haltenut 53 gegen die Vorsprünge 57, 57 des Sockels bzw. Fußes 52 der Kanüle 13 und zieht somit automatisch die Kanüle 13 von dem Innenkegelabschnitt 50 des Adapters 12 ab. Es ist somit ein Lösen der Kanüle 13 in sicherer Art und Weise möglich, da die Kanüle 13 selbst nicht in die Hand genommen werden muß. Allein durch Abschrauben der Aufnahmemutter 54 wird die Kanüle 13 vom Adapter 12 gelöst.

## Patentansprüche

1. Spritze mit
einem Grundkörper (2), der einen Spritzenzylinder (3) mit einem ausspritzseitigen Ende (4) aufweist, und
einer Kolbenstange (5), deren vorderer Endabschnitt (6) in dem Spritzenzylinder (3) entlang einer Verschieberichtung (P1) verschiebbar angeordnet ist, wobei der Grundkörper (2) einen vorderen Anschlag (15) für die Kolbenstange (5), bei der der Abstand zwischen dem vorderen Endabschnitt (6) und dem ausspritzseitigen Ende (4) des Spritzenzylinders (3) minimal ist, aufweist,
wobei die Spritze (1) ein Einstellelement (20) umfaßt, das einen hinteren Anschlag (21) für die Kolbenstange (5), bei der der Abstand zwischen dem vorderen Endabschnitt (6) und dem ausspritzseitigen Ende (4) des Spritzenzylinders (3) maximal ist, aufweist,
wobei das Einstellelement (20) relativ zum Grundkörper (2) bewegbar und in zumindest zwei vorgegebenen Einstellpositionen, in denen die Position des hinteren Anschlags (21) in Verschieberichtung (P1) verschieden ist, lösbar mit dem Grundkörper (2) verbindbar ist, so dass durch Wahl einer der Einstellpositionen für das Einstellelement (20) der maximale Hub der Kolbenstange (5) einstellbar ist,
und wobei das Einstellelement (20) ein federndes Druckstück (31, 40) aufweist und der Grundkörper (2) in jeder vorgegebenen Einstellposition eine Rastvertiefung (36) für das federnde Druckstück (31, 40) aufweist,
**dadurch gekennzeichnet, dass**
der Grundkörper (2) eine Führungsrille (34) für das federnde Druckstück (31, 40) aufweist, die sich entlang der Verschieberichtung (P1) erstreckt und in der das federnde Druckstück (31, 40) beim Verschieben des Einstellelementes (20) relativ zum Grundkörper (2) entlang der Verschieberichtung (P1) geführt ist,
dass für jede vorgegebene Einstellposition eine Justierrille (35) von der Führungsrille (34) quer zur Führungsrille (34) abzweigt, in die das federnde Druckstück (31, 40) durch ein relatives Verdrehen von Einstellelement (20) und Grundkörper (2) bringbar ist, und dass jede Justierrille (35) eine der Rastvertiefungen (36) aufweist.

2. Spritze nach Anspruch 1, **dadurch gekennzeichnet, dass**
das Einstellelement (20) entlang der Verschieberichtung (P1) relativ zum Grundkörper (2) bewegbar ist.

3. Spritze nach einem der obigen Ansprüche, **dadurch gekennzeichnet, dass** das Einstellelement (20) hohlzylinderförmig mit einem Boden (22) ausgebildet ist und der Boden den hinteren Anschlag (21) bildet.

4. Spritze nach Anspruch 3, **dadurch gekennzeichnet, dass** der Boden eine Durchgangsbohrung (23) aufweist, durch die die Kolbenstange (5) läuft.

5. Spritze nach Anspruch 3 oder 4, **dadurch gekennzeichnet, dass** die Kolbenstange (5) einen seitlichen Vorsprung (26) aufweist, der an einer Innenseite (27) des Bodens (22) anliegt, wenn die Kolbenstange (5) am hinteren Anschlag (21) ist.

6. Spritze nach einem der obigen Ansprüche, **dadurch gekennzeichnet, dass** das Einstellelement (20) ein weiteres federndes Druckstück (31, 40) aufweist und dass der Grundkörper (2) in jeder vorgegebenen Einstellposition eine Rastvertiefung (36) für das weitere federnde Druckstück (31, 40) aufweist.

7. Spritze nach einem der obigen Ansprüche, **dadurch gekennzeichnet, dass** das Einstellelement (20) einen vorstehenden Stift (32) aufweist und der Grundkörper (2) eine Führungsrille (34') für den Stift (32) aufweist, wobei sich die Führungsrille (34') entlang der Verschieberichtung (P1) erstreckt und der Stift (32) beim Verschieben des Einstellelementes (20) relativ zum Grundkörper (2) entlang der Verschieberichtung (P1) geführt ist.

8. Spritze nach einem der obigen Ansprüche, **dadurch gekennzeichnet, dass** die Führungsrille(n) (34, 34') auf einer Außenseite des Grundkörpers (20) ausgebildet ist/sind.

9. Spritze nach Anspruch 7 oder 8, **dadurch gekennzeichnet, dass** das Einstellelement (20) hohlzylinderförmig ausgebildet ist und der Stift (32) von der Innenwandung des hohlzylinderförmigen Einstellelements (20) nach innen vorsteht.

10. Spritze nach Anspruch 7, **dadurch gekennzeichnet, dass** der Stift (32) und das federnde Druckstück (31) einander gegenüber liegen.

11. Spritze nach einem der obigen Ansprüche, **dadurch gekennzeichnet, dass** die Position des vorderen Anschlags (15) nicht änderbar ist.

## Claims

1. Syringe with
a base body (2), which has a syringe cylinder (3) with an injection-side end (4), and
a piston rod (5), the front end section (6) of which is arranged in the syringe cylinder (3) so that it can be displaced along a displacement direction (P1), wherein the base body (2) has a front stop (15) for the piston rod (5), in which the distance between the front end section (6) and the injection-side end (4) of the syringe cylinder (3) is minimal,
wherein the syringe (1) comprises an setting element (20) which has a rear stop (21) for the piston rod (5), in which the distance between the front end section (6) and the injection-side end (4) of the syringe cylinder (3) is maximum,
wherein the setting element (20) can be moved relative to the base body (2) and can be detachably connected with the base body (2) in at least two predetermined setting positions, in which the position of the rear stop (21) is different in the displacement direction (P1), so that the maximum stroke of the piston rod (5) can be set by selecting one of the setting positions for the setting element (20),
and wherein the setting element (20) has a resilient pressure piece (31, 40) and that the base body (2) has a latching recess (36) for the resilient pressure piece (31, 40) in each predetermined setting position,
**characterized in that**
the base body (2) has a guide groove (34) for the resilient pressure piece (31, 40), which guide groove extends along the displacement direction (P1) and in which the resilient pressure piece (31, 40) is guided when the setting element (20) is displaced relative to the base body (2) along the displacement direction (P1),
for each predetermined setting position, an setting groove (35) branches off from the guide groove (34) transversely to the guide groove (34), in which the resilient pressure piece (31, 40) can be moved by relative rotation of the setting element (20 ) and base body (2), and
that each setting groove (35) has a latching recess (36).

2. Syringe according to claim 1, **characterized in that** the setting element (20) can be moved along the displacement direction (P1) relative to the base body (2).

3. Syringe according to one of the above claims, **characterized in that** the setting element (20) is in the form of a hollow cylinder with a base (22) and the base forms the rear stop (21).

4. Syringe according to claim 3, **characterized in that** the base has a through hole (23) through which the piston rod (5) extends.

5. Syringe according to claim 3 or 4, **characterized in that** the piston rod (5) has a lateral projection (26) which bears against an inner side (27) of the base (22) when the piston rod (5) bears against the rear stop (21).

6. Syringe according to one of the above claims, **characterized in that** the setting element (20) has a further resilient pressure piece (31, 40) and that the base body (2) has a latching recess (36) for the further resilient pressure piece (31, 40) for each predetermined setting position.

7. Syringe according to one of the above claims, **characterized in that** the setting element (20) has a projecting pin (32) and the base body (2) has a guide groove (34') for the pin (32), the guide groove (34') extends along the direction of displacement (P1) and the pin (32) is guided along the direction of displacement (P1) when the setting element (20) is displaced relative to the base body (2).

8. Syringe according to one of the above claims, **characterized in that** the guide groove(s) (34, 34') is/are formed on an outside of the base body (20).

9. Syringe according to claim 7 or 8, **characterized in that** the setting element (20) is in the form of a hollow cylinder and the pin (32) projects inwards from the inner wall of the hollow-cylindrical setting element (20).

10. Syringe according to claim 7, **characterized in that** the pin (32) and the resilient pressure piece (31) lie opposite one another.

11. Syringe according to any of the above claims, **characterized in that** the position of the front stop (15) cannot be changed.

## Revendications

1. Seringue avec :
un corps de base (2) qui comporte un cylindre d'injection (3) avec une extrémité du côté d'éjection (4) ; et
une tige de piston (5) dont la section d'extrémité avant (6) est disposé de façon à pouvoir coulisser dans le cylindre d'injection (3) le long d'une direction de coulissement (P1), le corps de base (2) comportant une butée (15) avant pour la tige de piston (5) pour laquelle la distance est minimale entre la section d'extrémité avant (6) et l'extrémité du côté d'éjection (4) du cylindre d'injection (3) ;
la seringue (1) comprenant un élément de réglage (20) qui comporte une butée (21) arrière est maximale pour la tige de piston (5) pour laquelle la distance entre la section d'extrémité avant (6) et l'extrémité du côté d'éjection (4) du cylindre d'injection (3) ;
l'élément de réglage (20) est mobile par rapport au corps de base (2) et peut être relié au corps de base (2) dans au moins deux positions de réglage prédéfinies dans lesquelles la position de la butée (21) arrière est différente dans la direction de coulissement (P1), de sorte que la course maximale de la tige de piston (5) soit réglable par sélection d'une des positions de réglage pour l'élément de réglage (20) ; et
l'élément de réglage (20) comportant une pièce de pression (31, 40) à ressort et le corps de base (2) comportant dans chaque position de réglage prédéfinie un renfoncement d'arrêt (36) pour la pièce de pression (31, 40) à ressort ;
**caractérisée en ce que** :
le corps de base (2) comporte une rainure de guidage (34) pour la pièce de pression (31, 40) à ressort qui s'étend le long de la direction de coulissement (P1) et dans laquelle la pièce de pression (31, 40) à ressort est guidée lors du coulissement de l'élément de réglage (20) par rapport au corps de base (2) le long de la direction de coulissement (P1) ;
une rainure d'ajustement (35) prédéfinie est divisée par la rainure de guidage (34) transversalement à la rainure de guidage (34) pour chaque position de réglage prédéfinie, dans laquelle la pièce de pression (31, 40) à ressort peut être amenée par une torsion relative de l'élément de réglage (20) et du corps de base (2) et que chaque rainure d'ajustement (35) comporte un des renfoncements d'arrêt (36).

2. Seringue selon la revendication 1, **caractérisée en ce que** l'élément de réglage (20) est mobile le long de la direction de coulissement (P1) par rapport au corps de base (2).

3. Seringue selon l'une quelconque des revendications précédentes, **caractérisée en ce que** l'élément de réglage (20) est réalisé en forme de cylindre creux avec un plancher (22) et le plancher formant la butée (21) arrière.

4. Seringue selon la revendication 3, **caractérisée en ce que** le plancher comporte un alésage traversant (23) à travers lequel la tige de piston (5) passe.

5. Seringue selon la revendication 3 ou 4, **caractérisée en ce que** la tige de piston (5) comporte une saillie latérale (26) butant contre un côté intérieur (27) du plancher (22) lorsque la tige de piston (5) est contre la butée (21) arrière.

6. Seringue selon l'une quelconque des revendications précédentes, **caractérisée en ce que** l'élément de réglage (20) comporte une pièce de pression (31, 40) à ressort supplémentaire et que le corps de base (2) comporte un renfoncement d'arrêt (36) pour la pièce de pression (31, 40) à ressort supplémentaire dans chaque position de réglage prédéfinie.

7. Seringue selon l'une quelconque des revendications précédentes, **caractérisée en ce que** l'élément de réglage (20) comporte une tige (32) saillante et le corps de base (2) comporte une rainure de guidage (34') pour la tige (32), la rainure de guidage (34') s'étendant le long de la direction de coulissement (P1) et la tige (32) étant guidée lors du coulissement de l'élément de réglage (20) par rapport au corps de base (2) le long de la direction de coulissement (P1) .

8. Seringue selon l'une quelconque des revendications précédentes, **caractérisée en ce que** la ou les rainures de guidage (34, 34') est/sont réalisées sur un côté extérieur du corps de base (20).

9. Seringue selon la revendication 7 ou 8, **caractérisée en ce que** l'élément de réglage (20) est réalisée en forme de cylindre creux et que la tige (32) ressort vers l'intérieur de la paroi intérieure de l'élément de réglage (20) en forme de cylindre creux.

10. Seringue selon la revendication 7, **caractérisée en ce que** la tige (32) et la pièce de pression (31) à ressort sont placées face à face.

11. Seringue selon l'une quelconque des revendications précédentes, **caractérisée en ce que** la position de la butée (15) avant n'est pas modifiable.
